# EUROPEAN PATENT APPLICATION

(11) **EP 4 825 188 A1**
(43) Date of publication of application: **21.10.2026**
(21) Application number: 24903804.3
(22) Date of filing: 16.12.2024
(51) Int. Cl.: A23L 27/00, A23L 2/00, A23L 2/52, A23L 5/00, C12N 9/90, C12P 19/02

(54) **ALLULOSE AMOUNT INCREASING AGENT**

(30) Priority: 15.12.2023 JP 2023212545
(71) Applicant: Amano Enzyme Inc., Nagoya-shi Aichi 460-8630 (JP)
(72) Inventor: KURITANI Yuko, Kakamigahara-shi, Gifu 509-0109 (JP); HAYASHI Takuma, Kakamigahara-shi, Gifu 509-0109 (JP); NAGAKI Kakeru, Kakamigahara-shi, Gifu 509-0109 (JP)
(74) Representative: WP Thompson
(86) International application number: PCT/JP2024/044463
(87) International publication number: WO 2025/127151

(57) **Abstract**

[Problems] To provide a technology for increasing the amount of allulose in raw materials or fructose-containing foods and drinks.

[Solution] The present technology provides an allulose amount increasing agent comprising D-allulose-3-epimerase. The allulose amount increasing agent may further comprise a glucosyltransferase. The present technology also provides a method for producing an allulose-containing food or drink, and a method for increasing the amount of allulose in a raw material or a fructose-containing food or drink, which include a step of treating a raw material or a fructose-containing food or drink with D-allulose-3-epimerase.

## Description

### Technical Field

The present invention relates to an allulose amount increasing agent. In particular, the present invention relates to an allulose amount increasing agent using a specific enzyme, an allulose-containing food or drink in which the allulose amount increasing agent is used, a method for producing an allulose-containing food or drink, and a method for increasing the amount of allulose in a fructose-containing food or drink.

### Background Art

In recent years, in response to the social trend toward health consciousness, the food market has seen an increase in the development of products that claim to be low in calories. For example, fruit juice drinks, vegetable juice drinks, and mixed juice drinks are widely used as a source of fruit and vegetable intake due to their convenience, and are preferred by many consumers for the purpose of maintaining good health. However, these drinks contain sugars derived from fruits and vegetables, and from the viewpoint of reducing calories, it is desirable to reduce the sugar content as much as possible.

For example, Patent Literature 1 discloses a method for producing low-calorie juice, which is characterized by treating fruit juice with an ultrafiltration membrane, thereby retaining cloudy fruit juice, in which the sugar content of the fruit juice has been concentrated, in the membrane, and allowing clear fruit juice with reduced sugar content to pass through the membrane. Furthermore, Patent Literature 2 discloses a method for producing vegetable or fruit juice, in which lactic acid bacteria are added to vegetable or fruit juice, the lactic acid bacteria are grown, and the sugar concentration in the vegetable or fruit juice is reduced by lactic acid fermentation. However, for example, the technology of treating fruit juice with a membrane has the problem that the richness and thickness of the juice are lost due to the removal of sugars. Furthermore, the technology of growing lactic acid bacteria and reducing calories through lactic acid fermentation requires sterilization and alcohol removal processes, so a technology that makes subsequent processes easier is needed.

### Citation List

### Patent Literatures

Patent Literature 1: Japanese translation of PCT publication No. 2015-522294
Patent Literature 2: Japanese Patent Application Laid-Open No. 10-271980

### Summary of Invention

### Technical Problem

As mentioned above, technologies for reducing the calorie content of a food or drink are being developed, but these technologies are still in the development stage.

Then, the main objective of this technology is to provide a technology for increasing the amount of allulose in a raw material or a fructose-containing food or drink.

### Solution to Problem

The present technology first provides an allulose amount increasing agent containing D-allulose-3-epimerase.

The D-allulose-3-epimerase may be derived from Asaia krungthepensis.

The allulose amount increasing agent according to the present technology may further comprise a glucosyltransferase.

The present technology also provides an allulose-containing food or drink, in which the allulose amount increasing agent is used.

The present technology also provides a method for producing an allulose-containing food or drink, which includes a step of treating a raw material or a fructose-containing food or drink with D-allulose-3-epimerase.

The method for producing an allulose-containing food or drink according to the present technology may further include a step of treating the raw material or the fructose-containing food or drink with glucosyltransferase.

Furthermore, the present technology also provides a method for increasing the amount of allulose in a fructose-containing food or drink, which includes a step of treating a raw material or a fructose-containing food or drink with D-allulose-3-epimerase.

The method for increasing the amount of allulose in a fructose-containing food or drink according to the present technology may further include a step of treating the raw material or the fructose-containing food or drink with glucosyltransferase.

In addition, the present technology also provides D-allulose-3-epimerase with excellent allulose-generating ability.

The technical terms used in this technology will be explained below.

### <Amino acid notation>

The 20 types of amino acid residues in an amino acid sequence may be expressed by single-letter abbreviations.

That is, glycine (Gly) is sometimes represented as G, alanine (Ala) as A, valine (Val) as V, leucine (Leu) as L, isoleucine (Ile) as I, phenylalanine (Phe) as F, tyrosine (Tyr) as Y, tryptophan (Trp) as W, serine (Ser) as S, threonine (Thr) as T, cysteine (Cys) as C, methionine (Met) as M, aspartic acid (Asp) as D, glutamic acid (Glu) as E, asparagine (Asn) as N, glutamine (Gln) as Q, lysine (Lys) as K, arginine (Arg) as R, histidine (His) as H, and proline (Pro) as P.

### <Amino acid sequence>

In this technology, the amino acid sequence displayed has the N-terminus at the left end and the C-terminus at the right end.

### <Types of amino acids>

In the present technology, "nonpolar amino acids" include alanine, valine, leucine, isoleucine, proline, methionine, phenylalanine, and tryptophan. "Uncharged amino acids" include glycine, serine, threonine, cysteine, tyrosine, asparagine, and glutamine. "Acidic amino acids" include aspartic acid and glutamic acid. "Basic amino acids" include lysine, arginine, and histidine.

### <Sequence modifications (substitutions, additions, insertions, deletions, inversions, etc.)>

In the present technology, modifications of amino acid sequences and/or base sequences include not only artificial modifications (substitutions, additions, insertions, deletions, inversions, etc.), but also naturally occurring modifications (substitutions, additions, insertions, deletions, inversions, etc.), i.e., modifications in which the sequences were originally different.

### <Substitution>

In the present technology, "substitution" refers not only to cases where amino acid residue substitutions are artificially introduced, but also to cases where amino acid residue substitutions are introduced naturally, i.e., cases where the amino acid residues are originally different. In the present technology, the substitution of amino acid residues may be artificial or natural, but artificial substitution is preferred. The amino acid residue at the mutation point (the amino acid residue to be substituted) is expressed by a combination of the above-mentioned single letter representing the type of amino acid and a number representing the position of the amino acid. For example, if arginine at position 57 is substituted with glycine, it will be expressed as "R57G".

### <Separation/purification>

In the present technology, the term "separation" is used interchangeably with "purification". The term "separated" is used to distinguish from the native state, i.e., the state as it exists in nature. The artificial process of "separation" results in a "separated state" that is different from the natural state. The separated product is clearly and decisively different from the natural product itself.

### <Equivalent sequence>

In the present technology, an "equivalent sequence" refers to an amino acid sequence or a base sequence that is partially different from a reference amino acid sequence (in the present technology, SEQ ID NOs: 1, 3, 4, 5, 6, 7, 8, 16, 17, 20, 21, 22, or 23) or base sequence (in the present technology, SEQ ID NOs: 2, 9, 10, 11, 12, 13, 14, 15, 18, or 19), but the difference does not substantially affect the function of the protein (in the present technology, D-allulose-3-epimerase activity or glucosyltransferase activity). Therefore, in the present technology, an enzyme having a polypeptide chain consisting of an equivalent amino acid sequence, or an enzyme encoded by a polynucleotide consisting of an equivalent base sequence, exhibits D-allulose-3-epimerase activity or glucosyltransferase activity.

### Description of Embodiments

A preferred embodiment for carrying out the present technology will be described below. It should be noted that the embodiment described below is an example of a typical embodiment of the present technology, and the scope of the present technology should not be interpreted as being narrowed.

### 1. Allulose amount increasing agent

The allulose amount increasing agent according to the present technology contains D-allulose-3-epimerase. Glucosyltransferase, other enzymes, and other components may also be included as needed.

As shown in the Examples below, the present inventors have discovered that the amount of allulose in an allulose-containing food or drink produced can be increased by treating a raw material or a fructose-containing food or drink with D-allulose-3-epimerase during the production of the allulose-containing food or drink.

Each component that can be used in the present technology will be described in detail below.

### (1) D-allulose-3-epimerase

D-allulose-3-epimerase that can be used in the present technology is an enzyme that catalyzes the epimerization of D-fructose as a substrate to generate allulose, and is a type of ketose-3-epimerase. Furthermore, the D-allulose-3-epimerase that can be used in the present technology may be an enzyme that also has other actions, as long as it has D-allulose-3-epimerase activity. In this technology, by producing an allulose-containing food or drink using D-allulose-3-epimerase, the effect of increasing the amount of allulose can be achieved.

The origin of the D-allulose-3-epimerase that can be used in the present technology is not particularly limited, and examples include D-allulose-3-epimerase derived from microorganisms of the genus Asaia (e.g., Asaia krungthepensis, etc.), the genus Arthrobacter (e.g., Arthrobacter globiformis, etc.), and the genus Pseudomonas ( e.g., Pseudomonas cichorii, etc.). Preferably, D-allulose-3-epimerase derived from a microorganism of the genus Asaia is used, and more preferably, D-allulose-3-epimerase derived from Asaia krungthepensis is used.

Allulose is classified as a ketose and is also called psicose. They are also called rare sugars because they exist naturally but in small amounts. Allulose is about 70% as sweet as sucrose, but has one-tenth the calories of sucrose, and has been reported to have functions such as suppressing the rise in postprandial blood sugar levels and preventing obesity. Therefore, increasing the amount of allulose in a fructose-containing food or drink can achieve an effect of reducing calories.

Here, "D-allulose-3-epimerase derived from Asaia krungthepensis" means D-allulose-3-epimerase produced by a microorganism classified as Asaia krungthepensis (which may be a wild-type strain or a mutant strain), or D-allulose-3-epimerase obtained by genetic engineering techniques using the D-allulose-3-epimerase gene. Therefore, a recombinant produced by a host microorganism into which the D-allulose-3-epimerase gene obtained from Asaia krungthepensis (or a modified version of said gene) has been introduced also falls under the category of "D-allulose-3-epimerase derived from Asaia krungthepensis".

Strains of Asaia krungthepensis are not particularly limited as long as they do not impair the action and effect of the present technology, and strains that are generally available from biological genetic resource institutions and the like can be used.

In this technique, microorganisms that produce enzymes that can be used in the present technology may be cultured, if necessary. Examples of microorganisms that produce D-allulose-3-epimerase include microorganisms of the genus Asaia, and examples of microorganisms of the genus Asaia include Asaia krungthepensis. Microorganisms that can be used may be wild-type strains or mutant strains (for example, mutant strains induced by ultraviolet irradiation).

The culture conditions in the culture step can be appropriately set taking into consideration the properties of the microorganism to be cultured, etc. Microorganisms can be cultured in solid or liquid media. In addition, for industrial production, aeration and agitation culture can also be used.

As the nutrient source for the medium, substances necessary for the growth of microorganisms can be used as appropriate. The carbon source may be any assimilable carbon compound, such as glucose, sucrose, lactose, maltose, molasses, and pyruvic acid. The nitrogen source may be any assimilable nitrogen compound, such as peptone, meat extract, yeast extract, casein hydrolysate, and alkaline extract of soybean meal. In addition to carbon sources and nitrogen sources, for example, salts of phosphates, carbonates, sulfates, magnesium, calcium, potassium, iron, manganese, zinc, etc.; specific amino acids; specific vitamins, etc. may be used appropriately as needed.

The culture temperature may be appropriately set within a range in which the microorganism to be cultured can grow and the microorganism can produce the enzyme that can be used in the present technology. The culture temperature can be set, for example, to about 15°C to 37°C. The culture time may be, for example, about 12 to 48 hours. The timing for ending the culture step can be, for example, when the enzyme that can be used in the present technology reaches its maximum yield.

The D-allulose-3-epimerase that can be used in this technology can be prepared from the culture solution of the microorganism from which the above-mentioned D-allulose-3-epimerase is derived. A specific preparation method includes recovering D-allulose-3-epimerase from the culture solution or microbial bodies of the above-mentioned microorganisms. For example, when a D-allulose-3-epimerase-secreting microorganism is used, the microbial bodies can be recovered from the culture solution in advance by filtration, centrifugation, or the like, as necessary, and then the enzyme can be separated and/or purified. Furthermore, when a microorganism that does not secrete D-allulose-3-epimerase is used, the microbial bodies may be recovered from the culture solution in advance, if necessary, and then the microbial bodies may be disrupted by pressure treatment, ultrasonic treatment, or the like to extract the enzyme, which can then be separated and/or purified. As a method for separating and/or purifying the enzyme, any known protein separation and/or purification method can be used without any particular limitation, and examples thereof include centrifugation, UF concentration, salting out, and various chromatographic methods using ion exchange resins, etc. The separated and/or purified enzyme can be powdered by a drying method such as freeze-drying or vacuum drying, and can also be powdered using an appropriate excipient and/or drying aid in the drying method. The separated and/or purified enzyme can also be formulated as a liquid by adding an appropriate additive and sterilizing by filtration.

In the present technology, a specific example of D-allulose-3-epimerase is the D-allulose-3-epimerase derived from Asaia krungthepensis shown below. For example, the amino acid sequence of D-allulose-3-epimerase derived from wild-type Asaia krungthepensis is shown in SEQ ID NO: 1. Furthermore, examples of the base sequence of DNA encoding the amino acid sequence shown in SEQ ID NO: 1 include the base sequences shown in SEQ ID NOs: 2 and 15. The base sequence shown in SEQ ID NO: 15 is the base sequence of DNA encoding wild-type D-allulose-3-epimerase derived from Asaia krungthepensis, and the base sequence shown in SEQ ID NO: 2 is the base sequence shown in SEQ ID NO: 15 modified to include codons suitable for Escherichia coli. Therefore, the DNA in this technology can be appropriately designed by those skilled in the art using the base sequence shown in SEQ ID NO: 2 as a reference sequence.

As another specific example, the amino acid sequences of the modified D-allulose-3-epimerase disclosed in WO 2023/157936 are shown in SEQ ID NOs: 3 to 8. The base sequences of DNAs encoding the amino acid sequences shown in SEQ ID NOs: 3 to 8 are shown in SEQ ID NOs: 9 to 14. "D-allulose-3-epimerase" as used herein includes D-allulose-3-epimerases obtained by artificially substituting a portion of the amino acid sequence of the wild-type D-allulose-3-epimerase described above, as well as D-allulose-3-epimerases that have been naturally substituted to have such amino acid sequences. "D-allulose-3-epimerase" as used herein also includes the above-mentioned modified D-allulose-3-epimerase (also referred to as "mutant D-allulose-3-epimerase").

Furthermore, as other specific examples, the amino acid sequences of modified D-allulose-3-epimerases are shown in SEQ ID NOs: 16 (M22K) and 17 (G14R). The base sequences of DNA encoding the amino acid sequences shown in SEQ ID NOs: 16 and 17 are shown in SEQ ID NOs: 18 and 19.

The present technology also provides a polypeptide shown in any one of (1) to (4) below.
(1) A polypeptide consisting of an amino acid sequence containing the amino acid substitutions G14R and/or M22K in the amino acid sequence shown in SEQ ID NO: 1;
(2) A polypeptide having the amino acid sequence according to (1) above, in which one or several amino acid residues are substituted, added, inserted or deleted, and which has D-allulose-3-epimerase activity and retains the amino acid substitutions G14R and/or M22K; and
(3) A polypeptide having a sequence identity of 90% or more to the amino acid sequence described in (1) above, having D-allulose-3-epimerase activity, and retaining the amino acid substitutions G14R and/or M22K.
(4) A polypeptide comprising the amino acid substitution according to any one of (1) to (3) above, and further comprising one or more amino acid substitutions selected from the group consisting of A56E, N57H, R58G, and F111W.
   The present technology also provides a polypeptide shown in any one of (5) to (7) below.
(5) A polypeptide consisting of an amino acid sequence containing one or more amino acid substitutions selected from the group consisting of M22K, G14R, and F111W in the amino acid sequence shown in SEQ ID NO: 1;
(6) A polypeptide having the amino acid sequence according to (1) above, in which one or several amino acid residues are substituted, added, inserted or deleted, and which has D-allulose-3-epimerase activity and retains one or more amino acid substitutions selected from the group consisting of M22K, G14R, and F111W; and
(7) A polypeptide having a sequence identity of 90% or more to the amino acid sequence described in (1) above, having D-allulose-3-epimerase activity, and retaining one or more amino acid substitutions selected from the group consisting of M22K, G14R, and F111W.

The amino acid substitutions in (5) above include single substitutions of M22K only, G14R only, or F111W only; double substitutions of M22K and G14R, M22K and F111W, or G14R and F111W; and triple substitutions of M22K, G14R, and F111W.

In the polypeptides (2) and (6) above, the amino acid modification introduced may include only one type of modification from substitution, addition, insertion, and deletion (e.g., substitution only, etc.), or may include two or more types of modifications (e.g., substitution and insertion, etc.). In the polypeptides (2) and (6) above, the number of amino acid differences at any difference site may be one or several, for example, 1 to 80, preferably 1 to 70, 1 to 60, 1 to 50, 1 to 40, or 1 to 30, more preferably 1 to 20, 1 to 10, 1 to 9, 1 to 8, 1 to 7, 1 to 6, 1 to 5, or 1 to 4, even more preferably 1 to 3, and particularly preferably 1 or 2, or 1. However, the amino acid substitutions (1) and (5) above are retained.

Furthermore, in the polypeptides (3) and (7) above, the sequence identity to the amino acid sequences of (1) and (5) above need only be 70% or more, but is preferably 80% or more, more preferably 85% or more, 90% or more, 95% or more, 96% or more, even more preferably 97% or more, still more preferably 98% or more, and particularly preferably 99% or more.

Here, in the polypeptides (3) and (7) above, the sequence identity to each of the amino acid sequences (1) and (5) above is the sequence identity calculated by comparing the polypeptides (3) and (7) above with the amino acid sequences (1) and (5) above. Furthermore, "sequence identity" refers to the value of amino acid sequence identity obtained by the bl2seq program (Tatiana A. Tatsusova, Thomas L. Madden, FEMS Microbiol. Lett., Vol. 174, pp. 247-250, 1999) of BLASTPACKAGE [sgi32 bit edition, Version 2.0.12; available from the National Center for Biotechnology Information (NCBI)]. The parameters are set as follows: Gap insertion cost value: 11, Gap extension cost value: 1.

The polypeptides (2) and (6) above, and (3) and (7) above are substantially identical to the polypeptides (1) and (5) above.

One or more selected from the group consisting of M22K, G14R, A56E, N57H, R58G, and F111W may be part of a larger protein (e.g., a fusion protein, etc.). Examples of sequences added to fusion proteins include sequences that are useful for purification, such as multiple histidine residues, and additional sequences that ensure stability during recombinant production.

When amino acid substitutions are introduced into the polypeptides (2) and (6) above, and (3) and (7) above relative to the polypeptides (1) and (5) above, a preferred embodiment of the introduced amino acid substitution is a conservative substitution. That is, examples of substitutions in the polypeptides (2) and (6) above, and (3) and (7) above include substitution with another nonpolar amino acid if the amino acid before substitution is a nonpolar amino acid, substitution with another uncharged amino acid if the amino acid before substitution is an uncharged amino acid, substitution with another acidic amino acid if the amino acid before substitution is an acidic amino acid, and substitution with another basic amino acid if the amino acid before substitution is a basic amino acid.

In the polypeptides (2) and (6) above and (3) and (7) above, "having D-allulose-3-epimerase activity" means that D-allulose-3-epimerase activity can be detected when measured using the "Method for measuring D-allulose-3-epimerase activity" in the Examples described below, and preferably means that the D-allulose-3-epimerase activity is equivalent to that of the polypeptides (1) and (5) above; specifically, when the D-allulose-3-epimerase activity of the polypeptides (1) and (5) above is taken as 1, the relative activity is approximately 0.8 to 1.2.

The polypeptides (1) to (7) above are referred to as D-allulose-3-epimerase derived from Asaia krungthepensis as long as they have D-allulose-3-epimerase activity.

The present technology also provides DNAs described in any of (1) to (3) below.
(1) DNA encoding a polypeptide set forth in either SEQ ID NO: 16 or 17;
(2) a polynucleotide consisting of the base sequence shown in SEQ ID NO: 18 or 19;
(3) DNA encoding a polypeptide having D-allulose-3-epimerase activity, which consists of a sequence equivalent to the base sequence shown in either SEQ ID NO: 18 or 19.

Examples of the base sequence of DNA encoding the amino acid sequence shown in SEQ ID NO: 1 include the base sequences shown in SEQ ID NO: 2 and SEQ ID NO: 15. The base sequence shown in SEQ ID NO: 15 is the base sequence of DNA encoding wild-type D-allulose-3-epimerase derived from Asaia krungthepensis, and the base sequence shown in SEQ ID NO: 2 is the base sequence shown in SEQ ID NO: 15 modified with codons suitable for Escherichia coli. Therefore, the DNA according to the present technology can be appropriately designed by those skilled in the art using the base sequence shown in SEQ ID NO: 2 as a reference sequence.

The base sequences shown in SEQ ID NOs: 18 and 19 are base sequences in which the codons have been changed to be suitable for Escherichia coli.

The DNA encoding D-allulose-3-epimerase is not limited to the above sequence, but may be DNA consisting of a sequence equivalent to the above sequence. Here, an equivalent sequence also includes DNA having a base sequence that is 70% or more, preferably 80% or more, more preferably 85% or more, even more preferably 90 % or more, 95% or more, 96% or more, 97% or more, even more preferably 98% or more, and particularly preferably 99% or more homologous to the base sequence of the DNA shown in (1) or (2) above, as long as it encodes a polypeptide having D-allulose-3-epimerase activity.

Here, DNA "homology" is calculated using publicly or commercially available software with algorithms that compare a reference sequence to a query sequence. Specifically, BLAST, FASTA, or GENETYX (Software Development Co., Ltd.) can be used, and these may be used with default parameters.

Furthermore, DNA encoding D-allulose-3-epimerase also includes base sequences in which several bases have been mutated by substitution, addition, insertion, or deletion corresponding to the substitution, addition, insertion, or deletion of the amino acid sequence described above, as long as the base sequence encodes a polypeptide having D-allulose-3-epimerase activity. Furthermore, a base sequence complementary to the DNA consisting of a base sequence encoding the above-mentioned polypeptide is also an equivalent sequence, and DNA that hybridizes with the DNA consisting of the base sequence under stringent conditions is also included in the DNA encoding D-allulose-3-epimerase, as long as it encodes a polypeptide having D-allulose-3-epimerase activity. The hybridization method can be carried out according to a conventional method. The same applies below.

DNA encoding D-allulose-3-epimerase can also be separated from microorganisms that produce the above-mentioned desired polypeptides. For example, the target DNA can be separated from the genome of Asaia krungthepensis by PCR or hybridization using the genomic DNA of the microorganism as a template, employing primers or probes designed from known amino acid sequence information taking into account gene degeneracy, or primers or probes designed based on known base sequence information.

The DNA encoding D-allulose-3-epimerase includes various types of DNA resulting from codon degeneracy. Various DNAs encoding the same amino acid sequence can be artificially produced easily using known genetic engineering techniques. For example, in the production of a protein by genetic engineering, if the codons used in the original gene encoding the target protein are infrequently used in the host, the amount of protein expressed may be low. In such cases, high expression of the target protein can be achieved by optimizing the codon usage frequency for the host without altering the encoded amino acid sequence.

Methods for introducing mutations into genes and artificially modifying amino acid sequences include known techniques such as the Kunkel method and the Gapped duplex method, and mutation introduction kits using site-directed mutagenesis, such as QuikChange Site-Directed Mutagenesis Kit (Stratagene), GeneArt Site-Directed Mutagenesis PLUS System (Invitrogen), and TaKaRa Site-Directed Mutagenesis System (Mutan-K, Mutan-Super Express Km, PrimeSTAR Mutagenesis Basal Kit, etc.: Takara Bio Inc.). The base sequence of the DNA and whether the obtained DNA encodes the target polypeptide can be confirmed by conventional methods.

In the present technology, an expression cassette or recombinant vector containing DNA encoding the above-mentioned desired polypeptide (hereinafter also referred to as the "expression cassette according to the present technology" or the "recombinant vector according to the present technology") can be obtained by linking a promoter and a terminator to DNA encoding D-allulose-3-epimerase, or by inserting the expression cassette according to the present technology or DNA encoding D-allulose-3-epimerase according to the present technology into an expression vector.

The expression cassette or recombinant vector according to the present technology may include, as control factors, a promoter and a terminator, as well as transcription elements such as an enhancer, a CCAAT box, a TATA box, or an SPI site, as necessary. These control factors need only be operably linked to the DNA of the present technology. The term "operably linked" means that the DNA of the present invention is linked to various control factors that regulate the DNA of the present technology in a state in which it can operate in a host cell.

Regarding the recombinant vector according to the present technology, an expression vector constructed for genetic recombination from a phage, a plasmid, or a virus that can autonomously replicate in a host is preferred. Such expression vectors are known, and examples of commercially available expression vectors include pQE-based vectors (manufactured by Qiagen K. K.), pDR540, pRIT2T (manufactured by GE Healthcare Biosciences Co., Ltd.), and pET-based vectors (manufactured by Merck KGaA). The expression vector may be used in an appropriate combination with the host cell. For example, when Escherichia coli is used as the host cell, examples include a combination of a pET-based vector and a DH5α Escherichia coli strain, a combination of a pET-based vector and a BL21 (DE3) Escherichia coli strain, or a combination of a pDR540 vector and a JM109 Escherichia coli strain.

A transformant (hereinafter also referred to as the "transformant according to the present technology") can be obtained by transforming a host using the expression cassette according to the present technology or the recombinant vector according to the present technology.

The host used for producing a transformant is not particularly limited as long as it allows a gene to be introduced, the expression cassette or recombinant vector is stable, and it is capable of autonomous replication and is capable of expressing the traits of a gene containing the DNA according to the present technology. Suitable examples include bacteria belonging to the genus Escherichia, such as Escherichia coli, the genus Bacillus, such as Bacillus subtilis, the genus Pseudomonas, such as Pseudomonas putida, and the genus Asaia, such as Asaia krungthepensis; yeast, and the like. In addition, animal cells, insect cells, plant cells, etc. may also be used.

The transformant according to the present technology can be obtained by introducing an expression cassette according to the present technology or a recombinant vector according to the present technology into a host. The location where the DNA according to the present technology is introduced is not particularly limited as long as the target gene can be expressed, and it may be on a plasmid or on the genome. Methods for introducing the expression cassette or recombinant vector according to the present technology include, for example, recombinant vector methods and genome editing methods. The conditions for introducing the expression cassette or recombinant vector into the host may be appropriately determined depending on the type of host, etc. When the host is a bacterium, examples of methods include a method using competent cells treated with calcium ions, and electroporation. When the host is yeast, examples of methods include electroporation, the spheroplast method, and the lithium acetate method. When the host is an animal cell, examples of the method include electroporation, calcium phosphate method, and lipofection method. When the host is an insect cell, examples of the method include the calcium phosphate method, lipofection method, and electroporation method. When the host is a plant cell, examples of the method include electroporation, Agrobacterium method, particle gun method, PEG method, etc.

Whether or not the expression cassette according to the present technology or the recombinant vector according to the present technology has been incorporated into the host can be confirmed by PCR, Southern hybridization, Northern hybridization, or the like. When confirming by PCR whether or not the expression cassette according to the present technology or the recombinant vector according to the present technology has been incorporated into a host, for example, genomic DNA, the expression cassette, or the recombinant vector may be separated and purified from the transformant.

When the host is a bacterium, for example, the expression cassette or recombinant vector is separated and purified based on a lysate obtained by lysing the bacterium. The method of lysis involves, for example, treatment with a lytic enzyme such as lysozyme, and if necessary, the use of proteases and other enzymes, as well as surfactants such as sodium lauryl sulfate (SDS), in combination. Additionally, physical disruption methods such as freeze-thawing and French press treatment may be combined. Separation and purification of DNA from the lysate can be carried out by, for example, deproteinization treatment using phenol treatment and protease treatment, ribonuclease treatment, alcohol precipitation treatment, and an appropriate combination of commercially available kits.

DNA can be cleaved according to a conventional method, for example, by treatment with a restriction enzyme. As the restriction enzyme, for example, a type II restriction enzyme that acts on a specific nucleotide sequence can be used. The DNA can be ligated to the expression cassette or expression vector using, for example, DNA ligase. Thereafter, PCR is carried out using the separated and purified DNA as a template and primers specific to the DNA according to the present technology. The amplified product obtained by PCR is subjected to agarose gel electrophoresis, polyacrylamide gel electrophoresis, capillary electrophoresis, or the like, and stained with ethidium bromide and SYBR Green solution, or the like, and the amplified product is detected as a band, thereby confirming that the transformation has been completed.

Alternatively, PCR can be carried out using primers that have been previously labeled with a fluorescent dye or the like, and the amplified product can be detected. Furthermore, a method may be employed in which the amplification product is bound to a solid phase such as a microplate, and the amplification product is confirmed by fluorescence, enzyme reaction, or the like.

Furthermore, the present technology encompasses a method for producing a polypeptide, which comprises culturing the above-mentioned transformant.

The culture conditions for the step of culturing the transformant may be appropriately set taking into consideration the nutritional and physiological properties of the transformant, and preferably include liquid culture. In addition, for industrial production, it is preferable to employ aeration and agitation culture. As the nutrient source for the medium, those necessary for the growth of the transformant are used. The carbon source may be any assimilable carbon compound, such as glucose, sucrose, lactose, maltose, molasses, and pyruvic acid. The nitrogen source may be any assimilable nitrogen compound, such as peptone, meat extract, yeast extract, casein hydrolysate, and alkaline extract of soybean meal. In addition to carbon sources and nitrogen sources, for example, salts of phosphates, carbonates, sulfates, magnesium, calcium, potassium, iron, manganese, zinc, etc.; specific amino acids; specific vitamins, etc. may be used appropriately as needed.

The culture temperature is appropriately set within a range in which the transformant can grow and produce the desired polypeptide. For example, the culture temperature is preferably about 15°C to 37°C. The cultivation may be completed at an appropriate time when the polypeptide reaches its maximum yield, and the cultivation time is usually about 12 to 48 hours.

After the step of culturing the transformant, the culture solution is subjected to a method such as centrifugation, and the culture supernatant and/or microbial bodies are recovered. The microbial bodies are treated with a mechanical method such as ultrasonication or a French press, or with a lytic enzyme such as lysozyme, and then solubilized, if necessary, with an enzyme such as protease or a surfactant such as sodium lauryl sulfate (SDS), to obtain a water-soluble fraction containing the desired oxygenase. Furthermore, by selecting an appropriate expression cassette or expression vector and host, the expressed polypeptide can be secreted into the culture solution.

The water-soluble fraction containing the polypeptide obtained as described above may be subjected to a purification treatment as it is, or the polypeptide in the water-soluble fraction may be concentrated and then subjected to a purification treatment. The concentration can be carried out by, for example, vacuum concentration, membrane concentration, salting out treatment, fractional precipitation using a hydrophilic organic solvent (e.g., methanol, ethanol, acetone, etc.), or the like. The purification treatment can be carried out by appropriately combining methods such as gel filtration, adsorption chromatography, ion exchange chromatography, and affinity chromatography. The purified desired polypeptide may be powdered by freeze-drying, vacuum drying, spray drying, or the like, if necessary.

Furthermore, modifications such as the addition of sugar chains and/or lipids, or N-terminal or C-terminal processing may be carried out. The above-mentioned modifications make it possible to simplify the extraction and purification of the recombinant protein, or to add biological functions.

When Asaia krungthepensis has been caused to produce the D-allulose-3-epimerase represented by the above-mentioned desired polypeptide due to mutation, the D-allulose-3-epimerase of the present invention can be produced by culturing the Asaia krungthepensis, recovering it from the culture solution or microbial bodies, and purifying it.

The content of D-allulose-3-epimerase in the allulose amount increasing agent according to the present technology can be freely set as long as it does not impair the effect of the present technology. The lower limit of D-allulose-3-epimerase can be set, for example, to 0.0003 U or more per 1 g of fructose used in the production of an allulose-containing food or drink. From the viewpoint of further enhancing the allulose-increasing effect, it can be set to preferably 0.0015 U or more, more preferably 0.003 U or more, 0.03 U or more, 0.3 U or more, 1 U or more, 10 U or more, 20 U or more, 30 U or more, 40 U or more, 50 U or more, 60 U or more, 70 U or more, even more preferably 70 U or more, and even more preferably 140 U or more.

The upper limit of the content of D-allulose-3-epimerase in the allulose amount increasing agent according to the present technology is not particularly limited as long as it does not impair the effect of the present technology, but can be set to, for example, 10,000 U or less, 9,000 U or less, 8,000 U or less, 7,000 U or less, 6,000 U or less, 5,000 U or less, 4,000 U or less, 3,000 U or less, 2,000 U or less, 1,000 U or less, 900 U or less, 800 U or less, 700 U or less, 600 U or less, 500 U or less, 400 U or less, 315 U or less, 300 U or less, 250 U or less, 200 U or less, or 180 U or less per 1 g of fructose used in the production of an allulose-containing food or drink.

In this technology, regarding the activity of D-allulose-3-epimerase, the enzyme amount that generates 1 mmol of allulose per minute when acted with a reaction solution containing 100 mM Tris-HCl buffer (1 mM CoCl₂) pH 7.5 and 50 (v/w)% fructose at 50°C for 1 hour is defined as 1 unit (1U).

### (2) Glucosyltransferase

Preferably, the allulose amount increasing agent according to the present technology further comprises a glucosyltransferase. By further including glucosyltransferase, the effect of increasing allulose can be expected to be even greater.

The glucosyltransferase that can be used in the present technology is an enzyme that hydrolyzes sucrose to generate one or more sugars selected from α-1,3 glucan and leucrose. The α-1,3 glucans that can be generated by the glucosyltransferase according to the present technology include insoluble α-1,3 glucans and soluble α-1,3 glucans. Furthermore, the insoluble α-1,3 glucan that can be generated by the glucosyltransferase according to the present technology contains saccharides with seven or more sugars. Soluble α-1,3 glucans that can be generated by the glucosyltransferase according to the present technology include nigerooligosaccharides such as nigerose, nigerotriose, nigerotetraose, nigeropentaose, and nigerohexaose. Note that the glucosyltransferase that can be used in the present technology may be an enzyme that also has other actions, as long as it has glucosyltransferase activity.

The origin of the glucosyltransferase that can be used in the present technology is not particularly limited, but examples include glucosyltransferases derived from bacteria (e.g., the genus Streptococcus), filamentous fungi (e.g., the genus Aspergillus), etc. Preferably, glucosyltransferase derived from a microorganism of the genus Streptococcus is used, and more preferably, glucosyltransferase derived from Streptococcus thermophilus is used.

Here, "glucosyltransferase derived from Streptococcus thermophilus" means a glucosyltransferase produced by a microorganism classified as Streptococcus thermophilus (whether a wild-type strain or a mutant strain), or a glucosyltransferase obtained by genetic engineering techniques using a glucosyltransferase gene. Therefore, a recombinant produced by a host microorganism into which a glucosyltransferase gene obtained from Streptococcus thermophilus (or a gene obtained by modifying said gene) has been introduced also falls under the category of "glucosyltransferase derived from Streptococcus thermophilus".

Streptococcus thermophilus is not particularly limited as long as it does not impair the action and effect of the present technology, and strains that are generally available from biological genetic resource institutions and the like can be used.

In this technique, microorganisms that produce enzymes that can be used in the present technology may be cultured, if necessary. Examples of microorganisms that produce glucosyltransferase include microorganisms of the genus Streptococcus, and examples of microorganisms of the genus Streptococcus include Streptococcus thermophilus. Microorganisms that can be used may be wild-type strains or mutant strains (for example, mutant strains induced by ultraviolet irradiation).

The culture conditions in the culture step can be appropriately set taking into consideration the properties of the microorganism to be cultured, etc. Microorganisms can be cultured in solid or liquid media. In addition, for industrial production, aeration and agitation culture can also be used.

As the nutrient source for the medium, substances necessary for the growth of microorganisms can be used as appropriate. The carbon source may be any assimilable carbon compound, such as glucose, sucrose, lactose, maltose, molasses, and pyruvic acid. The nitrogen source may be any assimilable nitrogen compound, such as peptone, meat extract, yeast extract, casein hydrolysate, and alkaline extract of soybean meal. In addition to carbon sources and nitrogen sources, for example, salts of phosphates, carbonates, sulfates, magnesium, calcium, potassium, iron, manganese, zinc, etc.; specific amino acids; specific vitamins, etc. may be used appropriately as needed.

The culture temperature may be appropriately set within a range in which the microorganism to be cultured can grow and the microorganism can produce the enzyme that can be used in the present technology. The culture temperature can be set, for example, to about 15°C to 37°C. The culture time may be, for example, about 12 to 48 hours. The timing for ending the culture step can be, for example, when the enzyme that can be used in the present technology reaches its maximum yield.

The glucosyltransferase that can be used in this technology can be prepared from the culture solution of the microorganism from which the glucosyltransferase is derived. A specific preparation method includes recovering glucosyltransferase from the culture solution or microbial bodies of the above-mentioned microorganism. For example, when a glucosyltransferase-secreting microorganism is used, the microbial bodies may be recovered from the culture solution in advance by filtration, centrifugation, or the like, as necessary, and the enzyme may then be separated and/or purified. Furthermore, when a non-glucosyltransferase-secreting microorganism is used, the microbial bodies may be recovered from the culture solution in advance, if necessary, and then disrupted by pressure treatment, ultrasonic treatment, or the like to extract the enzyme, which can then be separated and/or purified. As a method for separating and/or purifying the enzyme, any known protein separation and/or purification method can be used without any particular limitation, and examples thereof include centrifugation, UF concentration, salting out, and various chromatographic methods using ion exchange resins, etc. The separated and/or purified enzyme can be powdered by a drying method such as freeze-drying or vacuum drying, and can also be powdered using an appropriate excipient and/or drying aid in the drying method. The separated and/or purified enzyme can also be formulated as a liquid by adding an appropriate additive and sterilizing by filtration.

In the present technology, specific examples of glucosyltransferase include the glucosyltransferase derived from Streptococcus thermophilus shown below. For example, the glucosyltransferase may include a polypeptide derived from Streptococcus thermophilus and consisting of the amino acid sequence shown in SEQ ID NO: 20. The amino acid sequence including the signal sequence and pro-sequence is shown in SEQ ID NO: 21. Another specific example is a glucosyltransferase comprising a polypeptide consisting of the amino acid sequence shown in SEQ ID NO: 22 derived from Streptococcus thermophilus. The amino acid sequence including the signal sequence and pro-sequence is shown in SEQ ID NO: 23.

The glucosyltransferase that can be used in the present technology may be not only the polypeptide shown in (1) below, but also a glucosyltransferase including the polypeptides shown in (2) to (4) below. Furthermore, the glucosyltransferase that can be used in the present technology may be an artificial protein that is artificially designed based on the amino acid sequences (1) to (4) described below.
(1) A polypeptide consisting of the amino acid sequence shown in SEQ ID NO: 20 or SEQ ID NO: 22,
(2) A polypeptide having one or more amino acid residues substituted, added, inserted, deleted, or inverted in the amino acid sequence shown in SEQ ID NO: 20 or SEQ ID NO: 22 and having glucosyltransferase activity;
(3) A polypeptide having a sequence identity of 93% or more to the amino acid sequence shown in SEQ ID NO: 20 and having glucosyltransferase activity, in the amino acid sequence shown in SEQ ID NO: 20; and
(4) A polypeptide having a sequence identity of 90% or more to the amino acid sequence shown in SEQ ID NO: 22 and having glucosyltransferase activity, in the amino acid sequence shown in SEQ ID NO: 22.

The lower limit of the glucosyltransferase content in the allulose amount increasing agent according to the present technology can be freely set as long as it does not impair the effect of the present technology. The content of glucosyltransferase can be set to, for example, 0.0001 U or more per 1 g of fructose used in the production of an allulose-containing food or drink. From the viewpoint of further enhancing the allulose-increasing effect, the content can be set to preferably 0.001 U or more, more preferably 0.01 U or more, 0.03 U or more, 0.05 U or more, 0.1 U or more, 0.5 U or more, 1 U or more, 5 U or more, even more preferably 7.5 U or more, and even more preferably 10 U or more.

The upper limit of the glucosyltransferase content in the allulose amount increasing agent of the present technology is not particularly limited as long as it does not impair the effect of the present technology, but can be set to, for example, 500 U or less, 400 U or less, 300 U or less, 200 U or less, 100 U or less, or 50 U or less per 1 g of fructose used in the production of an allulose-containing food or drink.

In this technology, regarding the activity of glucosyltransferase, the enzyme amount that hydrolyzes 1 µmol of sucrose per minute when treated at 30°C using 50 mM sucrose solution (50 mM potassium phosphate buffer, pH 7.0) as a substrate is defined as 1 unit (1U).

### (3) Other components

The allulose amount increasing agent according to the present technology can be used in combination with other components as long as the action and effect of the present technology are not impaired. Examples of other components that can be used include excipients, pH adjusters, colorants, flavoring agents, disintegrants, lubricants, stabilizers, enzymes, and other components that are commonly used in pharmaceutical formulations. Furthermore, components having known or future functions can also be used in combination depending on the purpose.

### 2. Allulose-containing food or drink

The allulose-containing food or drink according to the present technology will be described in detail below.

### (1) Raw materials

The raw materials that can be used for the allulose-containing food or drink or fructose-containing food or drink according to the present technology are not particularly limited in terms of origin, type, etc., as long as they do not impair the effect of the present technology, and can be freely selected depending on the desired food or drink, but raw materials containing fructose (fructose-containing raw materials) are preferred.

Specific examples include fruit juices from grape, peach, banana, apple, strawberry, melon, mango, plum, cherry, orange, Satsuma mandarin, lemon, grapefruit, lime, mandarin, yuzu, tangerine, temple orange, tangelo, calamansi, persimmon, blueberry, pineapple, papaya, lychee, acerola, pear, European pear, oriental apricot, guava, prune, apricot, etc. Other examples include vegetable juices from carrots, tomatoes, bell peppers, paprika, Japanese mustard spinach, kale, broccoli, spinach, asparagus, daikon radish, Chinese cabbage, celery, cabbage, beets, onions, lettuce, parsley, watercress, pumpkin, watermelon, corn, etc. The fruit juice and/or vegetable juice may be used alone or in combination of two or more.

### (2) Fructose-containing food or drink

In the present technology, examples of the fructose-containing food or drink include fructose-containing foods or drinks produced by processing or cooking the above-mentioned raw materials. Examples of the fructose-containing foods or drinks include one or more selected from the group consisting of fruit juice drinks, vegetable juice drinks, and mixed juice drinks. Preferred examples include fruit juice drinks and vegetable juice drinks, and more preferred examples include apple juice drinks and carrot juice drinks.

In this technology, raw materials or fructose-containing food or drink are the target of the allulose amount increasing agent, and as described below, the allulose amount increasing agent of this technology reduces the amount of fructose in the raw materials or fructose-containing food or drink and increases the amount of allulose.

### (3) Allulose-containing food or drink

The allulose-containing food or drink according to the present technology is a food or drink produced by using an allulose amount increasing agent in the above-mentioned raw material or fructose-containing food or drink.

The amount of sugars contained in the allulose-containing food or drink of the present technology is not particularly limited, but it is preferable that the amount of fructose in the raw material or the raw material of the fructose-containing food or drink is reduced and the amount of allulose is increased. The content of fructose (also called "fruit sugar") contained in the raw material or fructose-containing food or drink that can be used with this technology can be freely set as long as the action and effect of the present technology are not impaired. The content of fructose is, for example, 1% by weight or more, 2% by weight or more, 3% by weight or more, 4% by weight or more, 5% by weight or more, or 6% by weight or more.

The sugar composition of allulose contained in the allulose-containing food or drink according to the present technology is, for example, 1% or more, preferably 1.5% or more, more preferably 5% or more, and even more preferably 7.5% or more. The upper limit of the sugar composition of allulose contained in the allulose-containing food or drink according to the present technology is not particularly limited, and is, for example, 30% or less, 25% or less, 20% or less, or 15% or less.

The sugar composition of fructose contained in the allulose-containing food or drink according to the present technology is, for example, 80% or less, preferably 70% or less, more preferably 65% or less, and even more preferably 50% or less. The lower limit of the fructose sugar composition contained in the allulose-containing food or drink according to the present technology is not particularly limited, and is, for example, 20% or more, 30% or more, or 40% or more.

Specific examples of the allulose-containing food or drink according to the present technology include one or more types selected from the group consisting of fruit juice drinks, vegetable juice drinks, and mixed juice drinks. Preferred examples include fruit juice drinks and vegetable juice drinks, and more preferred examples include apple juice drinks and carrot juice drinks.

### (4) Other

The allulose amount increasing agent according to the present technology can also be used in plant-derived molasses. Examples of plant-derived molasses sources include honey, agave syrup, and maple syrup.

In the present technology, the properties of the raw material or fructose-containing food or drink when subjected to various enzyme treatments are not particularly limited as long as they do not impair the effect of the present technology, but are preferably liquid or paste-like, and more preferably liquid. When raw materials having skin are used, either peeled or unpeeled raw materials can be used.

### 3. Method for producing allulose-containing food or drink, and method for increasing the amount of allulose in fructose-containing food or drink

The method for producing an allulose-containing food or drink and the method for increasing the amount of allulose in a fructose-containing food or drink according to the present technology are methods that include a step of treating a raw material or a fructose-containing food or drink with D-allulose-3-epimerase (also referred to as the "enzyme treatment step (I)"). In addition, the method for producing an allulose-containing food or drink and the method for increasing the amount of allulose in a fructose-containing food or drink according to the present technology may further include a step of treating the raw material or the fructose-containing food or drink with glucosyltransferase (also referred to as the "enzyme treatment step (II)"). Furthermore, depending on the type of raw material or fructose-containing food or drink or the like, it is also possible to carry out general food or drink producing steps and recovery steps before, after, or simultaneously with each process, as long as the effect of the present technology is not impaired. Each step will be described in detail below.

### (1) Enzyme treatment step (I)

The enzyme treatment step (I) is a step of treating a raw material or a fructose-containing food or drink with D-allulose-3-epimerase (including a step of treating fructose with any of the polypeptides (1) to (3) described above). If necessary, other enzymes may be used for treatment. These enzymes may be used alone or in combination of two or more. When two or more enzymes are used in combination, the two or more enzymes may be added simultaneously or separately to the raw material or the fructose-containing food or drink. When two or more enzymes are added separately, the order in which they are added is not particularly limited. Furthermore, the details and amount of the enzyme to be added are the same as those of the enzyme used in "1. Allulose amount increasing agent" described above, so the explanation thereof will be omitted here.

The specific method for adding each enzyme is not particularly limited, and as long as the enzyme can be added to the raw material or fructose-containing food or drink, it can be added by any method and at any timing. Each enzyme can also be added in two or more separate doses.

The various conditions for the enzyme treatment step (I) can be freely set as long as they do not impair the effect of the present technology. For example, the pH, temperature, treatment time, etc. can be set depending on the physicochemical properties of the enzyme used, such as optimal pH, stable pH range, optimal temperature, and temperature stability. The optimum reaction conditions can be determined through preliminary experiments. The conditions for the treatment with an enzyme are exemplified below.

When D-allulose-3-epimerase is added as an enzyme to a raw material or a fructose-containing food or drink, the pH can be set to, for example, pH 2.0 to 13.0, preferably pH 2.5 to 12.0, and more preferably pH 3.0 to 8.0. The temperature can be set, for example, to 20°C to 90°C, preferably 30°C to 80°C, and more preferably 40°C to 70°C. The treatment time can be set, for example, to 10 minutes to 6 hours, preferably 30 minutes to 4 hours, and more preferably 1 hour to 3 hours.

### (2) Enzyme treatment step (II)

The enzyme treatment step (II) is a step of treating a raw material or a fructose-containing food or drink with glucosyltransferase. The details and amount of the enzyme to be added are the same as those of the enzyme used in "1. Allulose amount increasing agent" described above, so the explanation thereof will be omitted here.

The specific method for adding glucosyltransferase is not particularly limited, and as long as the enzyme can be added to the raw material or fructose-containing food or drink, it can be added by any method and at any timing. Also, the glucosyltransferase can be added in two or more separate doses.

The various conditions for the enzyme treatment step (II) can be freely set as long as they do not impair the effect of the present technology. For example, the pH, temperature, treatment time, etc. can be set depending on the physicochemical properties of the enzyme used, such as optimal pH, stable pH range, optimal temperature, and temperature stability. The optimum reaction conditions can be determined through preliminary experiments. Examples of conditions for treatment with glucosyltransferase are given below.

When glucosyltransferase is added as an enzyme to a raw material or a fructose-containing food or drink, the pH can be set to, for example, pH 2.0 to 9.0, preferably pH 2.5 to 8.0, more preferably pH 3.0 to 6.0. The temperature can be set, for example, to 10°C to 70°C, preferably 20°C to 50°C, and more preferably 25°C to 45°C. The treatment time can be set, for example, to 15 minutes to 120 hours, preferably 30 minutes to 72 hours, and more preferably 2 hours to 48 hours.

### (3) Raw material preparation step

The raw material preparation step is a step of preparing the enzymes (D-allulose-3-epimerase and/or glucosyltransferase), raw materials (including fructose) used in the production of food or drink, etc. The raw material may be prepared in the form of a composition mixed with other components, as long as the action and effect of the present technology are not impaired.

### (4) Enzyme inactivation step

The enzyme inactivation step is a step of inactivating the enzyme after at least the above-mentioned enzyme treatment step (I). When the enzyme treatment step (II) is carried out, the enzyme can be inactivated after the enzyme treatment step (II).

The enzyme inactivation step is not an essential step and can be carried out as needed. Furthermore, when a plurality of enzymes are used in the enzyme treatment step (I) or the enzyme treatment step (II), an enzyme inactivation step can be carried out between the treatments with the enzymes. Furthermore, for example, when multiple enzymes are used for treatment in the enzyme treatment step (I) or the enzyme treatment step (II), it is also possible to carry out an enzyme inactivation step during the enzyme treatment step (I) or the enzyme treatment step (II). Furthermore, an enzyme inactivation step may be carried out between the enzyme treatment step (I) and the enzyme treatment step (II).

The method for inactivating an enzyme is not particularly limited, and a general inactivation method may be used alone or in combination of two or more methods depending on the properties of the enzyme to be inactivated. For example, the enzyme may be inactivated by heating to a temperature equal to or higher than the enzyme inactivation temperature.

### (5) Recovery step

The recovery step is a step of recovering the allulose-containing food or drink obtained through the enzyme treatment step (I) and, if necessary, other steps. As for the specific recovery method, depending on the state and type of the obtained allulose-containing food or drink, a single recovery method used in the production of a general allulose-containing food or drink may be used, or two or more methods may be used in combination.

The method according to the present technology described above can be embodied as including the following steps A to E.
A: a step of preparing raw materials or fructose-containing food or drink
B: a step of treating the prepared raw material or fructose-containing food or drink with D-allulose-3-epimerase
C: a step of further treatment with glucosyltransferase
D: a step of inactivating enzyme
E: a step of recovering the produced allulose-containing food or drink

Steps C and D are not essential and can be performed as needed. Furthermore, step D can be carried out multiple times as necessary after step B, during step B, after step C, during step C, during step E, or after step E. Furthermore, in step D, the enzyme can be inactivated by heating or the like during the production process of an allulose-containing food or drink. In addition, when a plurality of enzymes are used, the enzymes can be inactivated between the treatments with the enzymes (for example, between step B and step C). For example, when a plurality of enzymes are used for treatments in step B and/or step C, step D can be carried out in the middle of step B and/or step C as appropriate. Furthermore, step B and step C may be carried out simultaneously, step B may be followed by step C, or step B may be carried out after step C.

In the present technology, the following configuration can be adopted.
[1] An allulose amount increasing agent comprising D-allulose-3-epimerase.
[2] The allulose amount increasing agent according to [1], wherein the D-allulose-3-epimerase is derived from Asaia krungthepensis.
[3] The allulose amount increasing agent according to [1] or [2], further comprising glucosyltransferase.
[4] A food or drink, in which the allulose amount increasing agent according to any one of [1] to [3] is used.
[5] A method for producing an allulose-containing food or drink, comprising a step of treating a raw material or a fructose-containing food or drink with D-allulose-3-epimerase.
[6] The method for producing an allulose-containing food or drink according to [5], further comprising a step of treating the raw material or the fructose-containing food or drink with glucosyltransferase.
[7] A method for increasing the amount of allulose in a fructose-containing food or drink, comprising a step of treating a raw material or a fructose-containing food or drink with D-allulose-3-epimerase.
[8] The method for increasing the amount of allulose in a fructose-containing food or drink according to [7], further comprising a step of treating the raw material or the fructose-containing food or drink with glucosyltransferase.
[9] A polypeptide shown in any one of the following (1) to (3):
   (1) A polypeptide consisting of an amino acid sequence containing the amino acid substitutions G14R and/or M22K in the amino acid sequence shown in SEQ ID NO: 1;
   (2) A polypeptide having the amino acid sequence according to (1) above, in which one or several amino acid residues are substituted, added, inserted or deleted, and which has D-allulose-3-epimerase activity and retains the amino acid substitutions G14R and/or M22K; and
   (3) A polypeptide having a sequence identity of 90% or more to the amino acid sequence described in (1) above, having D-allulose-3-epimerase activity, and retaining the amino acid substitutions G14R and/or M22K.
[10] The polypeptide of [9], further comprising one or more amino acid substitutions selected from the group consisting of A56E, N57H, R58G, and F111W.
[11] DNA shown in any one of (1) to (3) below:
   (1) DNA encoding the polypeptide according to [9],
   (2) a polynucleotide consisting of the base sequence shown in SEQ ID NO: 18 or 19; and
   (3) DNA encoding a polypeptide having D-allulose-3-epimerase activity, which consists of a sequence equivalent to the base sequence shown in either SEQ ID NO: 18 or 19.
[12] An expression cassette or recombinant vector comprising the DNA according to [11].
[13] A transformant obtained by transforming a host with the expression cassette or recombinant vector according to [12].
[14] A method for producing the polypeptide according to [9], comprising a step of culturing the transformant according to [13].
[15] A method for producing allulose, comprising a step of treating fructose with the polypeptide according to [9].

### EXAMPLES

The present technology will be described in further detail below based on examples. It should be noted that the examples described below are examples of typical examples of the present technology, and the scope of the present technology should not be interpreted as being narrowed.

### <Experimental Example 1>

### (1) Raw materials

The raw materials and enzymes used in Experimental Example 1 are shown in Table 1 below.

**[Table 1]**

| Name of substance | Product name | Supplier |
|---|---|---|
| Apple juice drink | Kagome apple juice for business use 100 mL (fructose content: 6.4%/100 mL) | Kagome Co., Ltd. |
| D-allulose-3-epimerase | Asaia Krungthepensis-derived D-allulose-3-epimerase (SEQ ID No.1) | Amano Enzyme Inc. |

### (2) Method for measuring D-allulose-3-epimerase activity

10 mL of the sample solution containing DAE was added to 90 mL of 111 mM Tris-HCl buffer (1.11 mM CoCl₂, pH 7.5) containing 50 (v/w)% fructose, and the reaction solution was treated at 50°C for 1 hour and then boiled at 100°C for 5 minutes. After being allowed to cool, fructose and allulose were detected by HPLC, and the enzyme activity was determined by defining the amount of enzyme that generates 1 mmol of allulose per minute as 1 U.

### (3) Experimental example

### (3-1) Method

In order to prepare apple juice drinks with various pH values using an apple juice drink (Brix 11, pH 3.75, manufactured by Kagome Co., Ltd.; the same applies hereinafter) as a base, 1N aqueous sodium hydroxide solution was added to the apple juice drink to prepare apple juice drinks with various pH values (pH 4.0, 5.0, 6.0, and 7.0). D-allulose-3-epimerase (having the amino acid sequence shown in SEQ ID NO: 1) was added to each apple juice (pH 4.0, 5.0, 6.0, and 7.0) at a concentration of 156 U per 1 g of fructose, and the enzyme reaction was carried out at 50°C for 2 hours. After the enzyme reaction, the apple juice drink was heated at 100°C for 10 minutes to inactivate the enzyme, thereby preparing each enzyme-treated apple juice drink.

### (3-2) Analysis of sugar composition

The contents of various sugars in each of the enzyme-treated apple juice drinks prepared in (3-1) above and the non-enzyme-treated apple juice drink (control; Control Example 1) were measured by the following procedure. 1 mL of the reaction solution (centrifugation supernatant) was filtered through a membrane, and the carbohydrate composition was analyzed by high performance liquid chromatography (hereinafter also referred to as "HPLC") under the conditions shown in Table 2 below. From the obtained sugar composition, the ratio of allulose, fructose, and glucose to the total sugar content (sugar composition) was calculated.

**[Table 2]**

| | |
|---|---|
| Column | MCI GEL^{™} CK08EC (8.0 I.D. mm X 300 mm) (manufactured by Mitsubishi Chemical Corp.) |
| Eluent | H₂O (Milli-Q) |
| Column temperature | 75°C |
| Detector | RI(RID-10A: manufactured by Shimadzu Corp.) |
| Injection amount | 20µL |
| Analysis time | 30min |
| Flow rate | 0.6 mL/min |

### (3-3) Results

The results are shown in Table 3 below.

**[Table 3]**

| | | | Control Example 1 | Examples | | | |
|---|---|---|---|---|---|---|---|
| | | | | 1 | 2 | 3 | 4 |
| Enzyme used | D-allulose-3-epimerase | U/g-fructose | - | 156 | 156 | 156 | 156 |
| pH | | | 3.75 | 4.0 | 5.0 | 6.0 | 7.0 |
| Sugar composition (%) | Allulose | | 0.00 | 0.00 | 1.50 | 7.40 | 12.70 |
| | Fructose | | 69.20 | 69.20 | 67.80 | 61.90 | 56.50 |
| | Glucose | | 30.80 | 30.80 | 30.70 | 30.70 | 30.80 |

### (3-4) Consideration

As shown in Table 3 above, a comparison of the non-enzyme-treated example (Control Example 1) and the enzyme-treated examples (Examples 1 to 4) shows that allulose was generated at pH 5.0 or higher. In other words, it was confirmed that an apple juice drink with increased allulose content could be obtained.

### <Experimental Example 2>

### (1) Raw materials

The raw materials and enzymes used in Experimental Example 2 are shown in Table 4 below.

**[Table 4]**

| Name of substance | Product name | Supplier |
|---|---|---|
| Carrot juice drink | Kagome Carrot Juice, High Beta-Carotene | Kagome Co., Ltd. |
| D-allulose-3-epimerase | Asaia Krungthepensis-derived D-allulose-3-epimerase (SEQ ID No. 1) | Amano Enzyme Inc. |
| Glucosyltransferase 1 | Streptococcus thermophilus-derived glucosyltransferase (SEQ ID No. 20) | Amano Enzyme Inc. |
| Glucosyltransferase 2 | Streptococcus thermophilus-derived glucosyltransferase (SEQ ID No. 22) | Amano Enzyme Inc. |

### (2) Method for measuring D-allulose-3-epimerase activity

The enzyme activity was determined in the same manner as in Experimental Example 1 above.

### (3) Method for measuring glucosyltransferase activity

As substrates, 1700 µL of 50 mM potassium phosphate buffer (pH 7.0), 100 µL of 1 M sucrose solution (50 mM (1.71%) (17.1 g/L)), and 200 µL of enzyme solution were added to a test tube, mixed, and reacted at 30°C for 1 hour. After the reaction, the amount of free fructose was measured using E-kit Liquid D-glucose/fructose (manufactured by J.K. International Inc.), and the activity was calculated as a relative value. The amount of enzyme required to hydrolyze 1 µmol of sucrose per minute when treated with 50 mM sucrose solution (50 mM phosphate buffer (pH 7.0)) as a substrate at 30°C was defined as 1 U, and the enzyme activity was determined.

### (4) Experimental example

### (4-1) Method

Using a carrot juice drink (pH 5.12, manufactured by Kagome Co., Ltd.; the same applies hereinafter) as a base, 35.3 U of D-allulose-3-epimerase (D-allulose-3-epimerase having the amino acid sequence shown in SEQ ID NO: 1) per 1 g of sucrose, and 12.8 U of glucosyltransferase 1 (glucosyltransferase having the amino acid sequence shown in SEQ ID NO: 20) or glucosyltransferase 2 (glucosyltransferase having the amino acid sequence shown in SEQ ID NO: 22) per 1 g of sucrose were added to the carrot juice drink, and after stirring by inversion, the mixture was allowed to react at 30°C for 24 hours.

### (4-2) Analysis of sugar composition

The contents of various sugars in each of the enzyme-treated carrot juice drinks prepared in (4-1) above and the non-enzyme-treated carrot juice drink (controls; Control Examples 2 and 3) were measured by the following procedure. 1 mL of the reaction solution (supernatant after centrifugation) was filtered through a membrane, and the carbohydrate composition was analyzed by HPLC under the conditions shown in Table 5 below. From the obtained sugar composition, the ratio of allulose, fructose, and glucose to the total sugar content (sugar composition) was calculated.

**[Table 5]**

| | |
|---|---|
| Column | Shodex HILICpak VG-50 4E (250 mm x 4.6 mm I.D., 5 µm) |
| Mobile phase | A) MilliQ, B) Acetonitrile |
| Column temperature | 45°C |
| Detector | ELSD-LT III (Shimadzu Corp.) |
| | Gain: Wide |
| | Filter: 4 sec. |
| | Drift tube temp.: 40°C |
| | Nebulizer gas: N₂ |
| | Gas pressure: 350kPa |
| Injection amount | 10 µL |
| Analysis time | 60 min |
| Flow rate | 1.0 mL/min |
| Time program | 88%B (0-12 min)→83.5%B(25 min)→50%B(50 min)→88%B(50.1-60 min) |

### (4-3) Results

The results are shown in Tables 6 and 7 below.

**[Table 6]**

| | | Control Example 2 | Example 5 | Reference Example 1 | Example 6 |
|---|---|---|---|---|---|
| Enzyme used (U/g-sucrose) | D-allulose-3-epimerase | - | 35.3 | - | 35.3 |
| | Glucosyltransferase 1 | - | - | 12.8 | 12.8 |
| Sugar composition (%) | Allulose | 0.0 | 2.8 | 0.0 | 8.2 |
| | Fructose | 7.3 | 5.1 | 32.7 | 23.6 |
| | Glucose | 11.1 | 11.2 | 18.7 | 18.4 |

**[Table 7]**

| | | Control Example 3 | Example 7 | Reference Example 2 | Example 8 |
|---|---|---|---|---|---|
| Enzyme used (U/g-sucrose) | D-allulose-3-epimerase | - | 35.3 | - | 35.3 |
| | Glucosyltransferase 2 | - | - | 12.8 | 12.8 |
| Sugar composition (%) | Allulose | 0.0 | 2.9 | 0.0 | 6.0 |
| | Fructose | 9.4 | 7.2 | 25.3 | 20.5 |
| | Glucose | 14.1 | 13.9 | 20.2 | 20.4 |

### (4-4) Consideration

As shown in Tables 6 and 7 above, when comparing the non-enzyme-treated examples (Control Examples 2 and 3) with the enzyme-treated examples, Examples 5 and 7, which were treated with D-allulose-3-epimerase only, produced carrot juice drinks with increased allulose content. On the other hand, although no increase in allulose was confirmed in either Reference Example 1, in which enzyme treatment was performed only with glucosyltransferase 1, or Reference Example 2, in which enzyme treatment was performed only with glucosyltransferase 2, a significant increase in allulose was confirmed in both Examples 6 and 8, in which enzyme treatment was performed together with D-allulose-3-epimerase.

### <Experimental Example 3>

### (1) Raw materials

The raw materials and enzymes used in Experimental Example 3 are shown in Table 8 below.

**[Table 8]**

| Name of substance | Product name | Supplier |
|---|---|---|
| Apple juice drink | Kagome apple juice for business use 100 mL (fructose content: 6.4%/100 mL) | Kagome Co., Ltd. |
| D-allulose-3-epimerase | Asaia Krungthepensis-derived D-allulose-3-epimerase (wild type: SEQ ID No.1) | Amano Enzyme Inc. |
| D-allulose-3-epimerase | Asaia Krungthepensis-derived D-allulose-3-epimerase (mutant 1: SEQ ID No.16) | Amano Enzyme Inc. |
| D-allulose-3-epimerase | Asaia Krungthepensis-derived D-allulose-3-epimerase (mutant 2: SEQ ID No.17) | Amano Enzyme Inc. |
| D-allulose-3-epimerase | Asaia Krungthepensis-derived D-allulose-3-epimerase (mutant 3: SEQ ID No.6) | Amano Enzyme Inc. |

### (2) Enzyme acquisition and identification

Using the full-length D-allulose-3-epimerase sequence (SEQ ID NO: 2) introduced into the pET24 vector as a template, PCR was carried out in a conventional method to introduce mutations.

The resulting amplification product was used to transform E. coli JM109 to obtain a gene expression vector. The D-allulose-3-epimerase sequence introduced into the gene expression vector was confirmed. The gene expression vector was introduced into E. coli BL21(DE3). The transformant was cultured in LB medium at 37°C for 3 hours with shaking. After that, the transformant was cultured at 25°C for 20 minutes with shaking, IPTG (final concentration 1 mM) was added, and the mixture was cultured at 25°C for 24 hours with shaking. The microbial bodies were recovered from the culture solution, suspended in 50 mM Tris-HCl buffer (pH 7.5), and then disrupted with a beads shocker. The mixture was centrifuged at 15,000 rpm for 10 minutes. The recovered centrifuged supernatant was used as an enzyme sample.

### (2) Measurement of D-allulose-3-epimerase activity

### Method

The enzyme activity was determined in the same manner as in Experimental Example 1 above.

### (3) Experimental example

### (3-1) Method

In order to prepare apple juice drinks with various pH values using an apple juice drink (Brix 11, pH 3.75, manufactured by Kagome Co., Ltd.; the same applies hereinafter) as a base, 1N sodium hydroxide solution was added to the apple juice drink to prepare apple juice drinks with various pH values (pH 4.0, 5.0, 6.0, and 7.0). D-allulose-3-epimerase (Variant 1 (M22K): D-allulose-3-epimerase having the amino acid sequence shown in SEQ ID NO: 16; Variant 2 (G14R): D-allulose-3-epimerase having the amino acid sequence shown in SEQ ID NO: 17; Variant 3 (F111W): D-allulose-3-epimerase having the amino acid sequence shown in SEQ ID NO: 6) was added to each apple juice drink (pH 4.0, 5.0, 6.0, and 7.0) at a concentration of 156 U per 1 g of fructose, and the enzyme reaction was carried out at 50°C for 2 hours. After the enzyme reaction, the apple juice drink was centrifuged at 15,000 G for 5 minutes to prepare each enzyme-treated apple juice drink.

### (3-2) Analysis of sugar composition

The sugar content of each of the enzyme-treated apple juice drinks prepared in (3-1) above was measured by the following procedure. 1 mL of the reaction solution (supernatant after centrifugation) was filtered through a membrane, and the carbohydrate composition was analyzed by HPLC under the conditions shown in Table 9 below. From the obtained sugar composition, the ratio of allulose, fructose, and glucose to the total sugar content (sugar composition) was calculated.

**[Table 9]**

| | |
|---|---|
| Column | MCI GEL^{™} CK08EC (8.0 I.D. mm X 300 mm) (manufactured by Mitsubishi Chemical Corp.) |
| Eluent | H₂O (Milli-Q) |
| Column temperature | 75°C |
| Detector | RI(RID-10A: manufactured by Shimadzu Corp.) |
| Injection amount | 20µL |
| Analysis time | 30min |
| Flow rate | 0.6 mL/min |

### (3-3) Results

The results are shown in Tables 10 and 11 below.

**[Table 10]**

| | | Example 7 | | | Example 8 | | |
|---|---|---|---|---|---|---|---|
| pH | | 4.0 | | | 5.0 | | |
| Enzyme used | | D-allulose-3-epimerase | | | D-allulose-3-epimerase | | |
| | | Mutant 1 | Mutant 2 | Mutant 3 | Mutant 1 | Mutant 2 | Mutant 3 |
| Enzyme addition amount (U/mL-fructose) | | 156 | 156 | 156 | 156 | 156 | 156 |
| Sugar composition (%) | Allulose | 0.1 | 0.0 | 0.0 | 3.7 | 5.1 | 2.1 |
| | Fructose | 68.3 | 68.3 | 68.5 | 64.7 | 63.3 | 66.3 |
| | Glucose | 31.5 | 31.6 | 31.5 | 31.6 | 31.7 | 31.6 |

**[Table 11]**

| | | Example 9 | | | Example 10 | | |
|---|---|---|---|---|---|---|---|
| pH | | 6.0 | | | 7.0 | | |
| Enzyme used | | D-allulose-3-epimerase | | | D-allulose-3-epimerase | | |
| | | Mutant 1 | Mutant 2 | Mutant 3 | Mutant 1 | Mutant 2 | Mutant 3 |
| Enzyme addition amount (U/mL-fructose) | | 156 | 156 | 156 | 156 | 156 | 156 |
| Sugar composition (%) | Allulose | 17.4 | 17.7 | 7.7 | 18.3 | 18.3 | 12.7 |
| | Fructose | 50.9 | 50.5 | 60.7 | 50.0 | 49.9 | 55.7 |
| | Glucose | 31.7 | 31.8 | 31.5 | 31.7 | 31.8 | 31.6 |

### (3-4) Consideration

As shown in Tables 10 and 11 above, when comparing the enzyme-treated examples (Examples 7 to 10), allulose was also generated in D-allulose-3-epimerase mutants 1 to 3 at a pH of 5.0 or higher. In particular, D-allulose-3-epimerase mutants 1 and 2 generated more allulose at pH 5.0 or higher. In other words, it was confirmed that an apple juice drink with increased allulose content could be obtained.

## Claims

1. An allulose amount increasing agent comprising D-allulose-3-epimerase.

2. The allulose amount increasing agent according to claim 1, wherein the D-allulose-3-epimerase is derived from Asaia krungthepensis.

3. The allulose amount increasing agent according to claim 1 further comprising a glucosyltransferase.

4. An allulose-containing food or drink, in which the allulose amount increasing agent according to any one of claims 1 to 3 is used.

5. A method for producing an allulose-containing food or drink, comprising a step of treating a raw material or a fructose-containing food or drink with D-allulose-3-epimerase.

6. The method for producing an allulose-containing food or drink according to claim 5, further comprising a step of treating the raw material or the fructose-containing food or drink with glucosyltransferase.

7. A method for increasing the amount of allulose in a fructose-containing food or drink, comprising a step of treating a raw material or a fructose-containing food or drink with D-allulose-3-epimerase.

8. The method for increasing the amount of allulose in a fructose-containing food or drink according to claim 7, further comprising a step of treating the raw material or the fructose-containing food or drink with glucosyltransferase.
